# EUROPÄISCHE PATENTSCHRIFT

(11) **EP 2 626 699 B1**
(45) Veröffentlichungstag und Bekanntmachung des Hinweises auf die Patenterteilung: **30.09.2020**
(21) Anmeldenummer: 13153612.0
(22) Anmeldetag: 01.02.2013
(51) Int. Cl.: G01N 29/024, G01N 29/032, G01N 29/22, G01N 33/38

(54) **Vorrichtung zur in situ Charakterisierung der Qualitätsparameter und/oder der Eigenschaften von anorganischen Bindemittelsystemen**
Apparatus for in situ characterization of quality parameters and/or the properties of inorganic binding agent systems
Dispositif destiné à la caractérisation in situ des paramètres de qualité et/ou des propriétés des systèmes de liant inorganiques

(30) Priorität: 10.02.2012 DE 102012101108; 08.03.2012 DE 102012101944
(43) Veröffentlichungstag der Anmeldung: 14.08.2013
(73) Patentinhaber: MF Instruments GmbH, 72461 Albstadt (DE)
(72) Erfinder: Feist, Manfred, 72461 Albstadt (DE)
(74) Vertreter: Patentanwälte und Rechtsanwalt Weiß, Arat & Partner mbB

(56) Entgegenhaltungen:
- WO-A1-2012/003980
- FR-A- 893 459
- Robert Long: "The improvement of ultrasonic apparatus for routine inspection of concrete", , 1. März 2000 (2000-03-01), XP055072902, Gefunden im Internet: URL:http://www3.imperial.ac.uk/pls/portall ive/docs/1/50545706.PDF [gefunden am 2013-07-25]
- Anne Jüngert: "Untersuchung von GFK-Bauteilen mit akustischen Verfahren am Beispiel der Rotorblätter von Windenergieanlagen", , 21. Juni 2010 (2010-06-21), XP055073032, Gefunden im Internet: URL:http://elib.uni-stuttgart.de/opus/voll texte/2010/5289/pdf/DissJuengert.pdf [gefunden am 2013-07-25]

## Beschreibung

Die Erfindung betrifft eine Vorrichtung zur in situ Charakterisierung der Qualitätsparameter und/oder der Eigenschaften von anorganischen Bindemittelsystemen, insbesondere von aushärtbaren Gemischen, wobei sich das Bindemittelsystem in einem Aufnahmeelement befindet, dem zumindest eine Wand mit einer Sonde zugeordnet ist, wobei zwischen dem Bindemittelsystem und der Sonde eine Kammer für ein Kontaktmaterial vorgesehen ist und eine Membran das Bindemittelsystem von dem Kontaktmaterial trennt, wobei die Membran bei einem Schwinden des Bindemittels diesem folgt.

### Stand der Technik

Für die Prüfung von Qualitätsparametern und/oder von Eigenschaften von anorganischen Bindemittelsystemen sind aus dem Stand der Technik verschiedene Prüfverfahren bekannt. Hierbei werden insbesondere zerstörungsfreie und zerstörende Prüfverfahren unterschieden. Für Beton ist beispielsweise ein zerstörungsfreies Prüfverfahren bekannt, bei welchem Ultraschall durch den Beton geschickt wird, um die Festigkeit des Betons zu ermitteln. Dieses Verfahren macht sich zu Nutze, dass eine Laufzeit des Schalls in Beton je nach Härte des Betons variiert.

Schwierigkeiten bei dieser Messung ergeben sich aber vor allem daraus, dass der Beton beim Aushärten schrumpft bzw. schwindet. Das bedeutet, dass hierdurch zwischen der Schall-Sonde und dem Beton eine Luftstrecke erzeugt und beim Schwinden auch vergrössert wird, wodurch diese Verfahren sehr fehlerhaft sind.

In der Veröffentlichung Robert Long: "The improvement of ultrasonic apparatus for routine inspection of concrete", 1. März 2000 (2000-03-01), XP055072902, URL: http://www.3.imperial.ac.uk/pls/portallife/docs/1/50545706.PDF wird ferner auf der Seite 183 ein System zur Überprüfung von Beton beschrieben, bei dem einerseits eine Sonde über eine Gummimatte auf dem Beton aufsitzt, andererseits eine Sonde/Empfänger über ein Klebemittel mit dem Beton verbunden ist.

Eine Vorrichtung der o.g. Art ist aus der DE 10 2010 060 068.7 bzw. WO 2012/003980 A1 bekannt. Dort wird auch ausgeführt, dass beim Schwinden des Bindemittelsystems die Membranen unter dem Druck des Gels folgen, so dass sie permanent in Kontakt mit dem Bindemittelsystem bleiben.

### Aufgabe der Erfindung

Aufgabe der Erfindung ist es, eine Vorrichtung der o. g. Art zu schaffen, mit der zerstörungsfrei in situ Charakterisierungen von anorganischen Bindemittelsystemen erfolgen können, welche noch zuverlässiger die Qualitätsparameter und die Eigenschaften ermitteln.

### Lösung der Aufgabe

Zur Lösung der Aufgabe führt, dass die Membran mit dem Bindemittelsystem durch Kleben verbunden ist.

Als Sonde kommt vor allem eine Schall-Sonde und hier eine Ultraschall-Sonde in Betracht, wobei nicht nur Schall-Laufzeitänderungen erfasst werden können, sondern auch Änderungen in der Schall-Energie und der Schall-Abschwächung oder im Frequenzspektrum. Die Erfindung soll aber nicht auf Schall-Sonden beschränkt sein, es sollen alle Sonden umfasst sein, welche ein Messmittel durch ein Bindemittelsystem schicken. Beispielsweise kann dies auch ein elektrisches Signal sein, dessen Frequenzspektrum sich je nach Dichte des Bindemittelsystems ändert. Hier soll der Erfindung keine Grenze gesetzt sein. Als Kontaktmaterial kommt vor allem ein solches in betracht, welches sich über ein längeren Zeitraum hinweg an ein Schrumpfen, Schwinden oder Ausdehnung des Bindemittelsystems anpassen kann. Hier bietet sich beispielsweise ein Gel an. Wichtig ist allein, dass dieses Gel mögliche Luftstrecken zwischen dem Bindemittelsystem und der Sonde ausgleicht. Das heisst, beim Schwinden des Bindemittels muss Gel nachfließen, beim Ausdehnen des Bindemittels muss Gel verdrängt werden können.

In der Praxis hat sich herausgestellt, dass ein Schwinden des Bindemittelsystems sogar dazu führen kann, dass eine Membran trotz dem Druck des Gels dem Bindemittelsystem nicht mehr folgen kann. Durch die vorliegende Erfindung wird jedoch vorgeschrieben, dass dieses Folgen unbedingt sein muss. Nur hierdurch wird ein Kontaktabbruch für beispielsweise Ultraschall, der von der Sonde ausgesendet wird, vermieden.

Die Membran wird erfindungsgemäß direkt mit dem Bindemittelsystem verbunden. Dies kann beispielsweise dadurch geschehen, dass die Membran selbst eine Adhäsionsschicht aufweist, die dafür sorgt, dass die Membran an dem Bindemittelsystem anhaftet, auch wenn dieses schwindet. Es ist aber auch möglich, auf die Membran selbst z.B. eine Klebefolie, insbesondere eine doppelseitige Klebefolie, aufzubringen, die dem Bindemittelsystem anhaftet und folgt.

### FIGURENBESCHREIBUNG

Nachfolgend wird die Erfindung anhand der beiliegenden Figuren kurz beschrieben, wobei die Figuren zeigen:
- **Figur 1**: eine Draufsicht auf eine erfindungsgemäße Vorrichtung zur in situ Charakterisierung der Qualitätsparameter und der Eigenschaften von anorganischen Bindemittelsystemen;
- **Figur 2**: eine Seitenansicht auf eine Wand der Vorrichtung gemäß Figur 1.

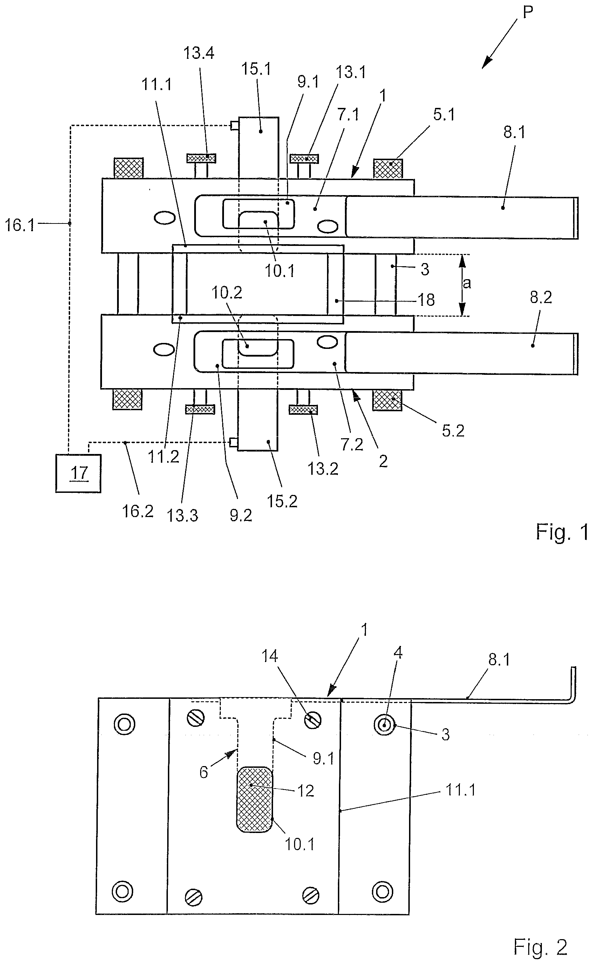

Eine erfindungsgemäße Vorrichtung P zur in situ Charakterisierung der Qualitätsparameter und der Eigenschaften von anorganischen Bindemittelsystemen weist gemäß Figur 1 zwei Wände 1 und 2 auf, die in einem Abstand a voneinander angeordnet sind. Dieser Abstand a wird von Distanzhülsen 3 bestimmt, die jeweils einem Schraubenbolzen 4 aufgeschoben sind. Vier derartige Schraubenbolzen 4 durchsetzen jeweils die Wände 1 und 2, wobei auf aus den Wänden 1 und 2 (nicht mehr dargestellte) herausragende Enden des Schraubenbolzens 4 Rändelmuttern 5.1 bzw. 5.2 aufgeschraubt sind.

Jede Wand 1 bzw. 2 besitzt eine in Figur 2 gestrichelt bzw. erkennbare Stufenausnehmung 6. Ein von oben gesehen erster Teil der Stufenausnehmung 6 ist als eine flache Führung 7.1 bzw. 7.2 für einen Schieber 8.1 bzw. 8.2 ausgebildet. Mit diesem Schieber 8.1 bzw. 8.2 kann die Stufenausnehmung 6 insgesamt verschlossen werden.

An die flache Führung 7.1 bzw. 7.2 schließt ein Trichterschacht 9.1 bzw. 9.2 an, der in eine ovale Kammer 10.1 bzw. 10.2 übergeht. Dies ovale Kammer 10.1 bzw. 10.2 durchbricht in dieser ovalen Form auch die Wand 1 bzw. 2 nach aussen hin und sogar eine Platte 11.1 bzw. 11.2, welche in die Wand 1 bzw. 2 eingelassen ist. Allerdings wird die Kammer 10.1 bzw. 10.2 von einer Membran 12 überdeckt, welche zwischen der Platte 11.1 bzw. 11.2 und der Wand 1 bzw. 2 eingespannt ist. Diese Membran 12 kann bei Bedarf auch ausgetauscht werden, wozu vier Rändelmuttern 13.1 - 13.4 gelöst werden, welche auf Schraubenbolzen 14 aufsitzen, die wiederum durch die Wand 1 bzw. 2 hindurchgeführt sind.

An die Kammer 10.1 bzw. 10.2 schließt eine Schall-Sonde 15.1 bzw.15.2 an, die über eine entsprechende Verbindung 16.1 bzw. 16.2 mit einer Auswerteeinheit 17 in Verbindung steht.

Zwischen den beiden Wänden 1 und 2 ist ein U-förmiges Aufnahmeelement 18 eingesetzt, welches bevorzugt aus einem Schaumstoff besteht. Dieses Aufnahmeelement 18 dient der Aufnahme eines zu untersuchenden Bindemittelsystems, weshalb es von der Festigkeit her geeignet sein muss, dieses Bindemittelsystem aufzunehmen und zu halten. Andererseits muss es elastisch genug sein, damit es abdichtend zwischen den beiden Wänden 1 und 2 eingespannt werden kann. Aus diesem Grunde muss auch eine Breite des Aufnahmeelementes 18 mit einer Länge der Distanzhülse 3 abgestimmt werden, sodass es zu einem geeigneten Einspannen des Aufnahmeelementes 18 zwischen den beiden Wänden 1 und 2 kommt.

Die Funktionsweise der vorliegenden Erfindung ist folgende:
Zunächst werden die beiden Wände 1 und 2 aufeinander festgelegt, um die Schall-Sonden 15.1 und 15.2 zu kalibrieren und die Auswerteeinheit 17 mit den entsprechenden Signalen zu füttern. Die beiden Kammern 10.1 und 10.2 sind mit Gel gefüllt und die entsprechenden Stufenausnehmungen 6.1 bzw. 6.2 durch die Schieber 8.1 bzw. 8.2 verschlossen.

Nach dieser Kalibrierung werden die beiden Wände 1 und 2 durch die entsprechenden Schraubenbolzen 4 verbunden und der Abstand a durch die Distanzhülsen 3 erzeugt. Dabei wird bereits das Aufnahmeelement 18 zwischen die beiden Wände 1 und 2 eingesetzt bzw. eingespannt.

Nunmehr kann ein zu untersuchendes Bindemittelsystem in das Aufnahmeelement 18 eingefüllt werden. Dieses Bindemittelsystem härtet aus, wobei nach vorgegebenen Zeitspannen Messungen mittels der Schall-Sonden 15.1 und 15.2 durchgeführt werden, um beispielsweise eine erreichte Druckfestigkeit des Bindemittelsystems in dem Aufnahmeelement 18 zu überprüfen. Im Rahmen der Erfindung ist es dabei wesentlich, dass ein Schwinden des Bindemittelsystems in dem Aufnahmeelement 18 durch Nachfließen von Gel ausgeglichen wird, welches die Membran 12.1 bzw. 12.2 gegen das schwindende Bindemittelsystem drückt. Der Trichterschacht 9.1 bzw. 9.2 dient dabei als Vorratsraum für das nachfließende Gel.

Die Membranen 12.1 bzw. 12.2 bestehen bevorzugt aus einem Kunststoff und besitzen eine Dicke von weniger als 2 mm, bevorzugt von weniger als 1 mm. Beim Schwinden des Bindemittelsystems folgen die Membranen unter dem Druck des Gels, sodass sie permanent in Kontakt mit dem Bindemittelsystem bleiben. Hierdurch wird eine Übertragung der Schall-Sonden 15.1 bzw. 15.2 nicht unterbrochen, das heißt es befindet sich weder Luft zwischen der Membran und dem zu untersuchenden Bindemittelsystems noch zwischen der Membran und dem Gel.

Das erfindungsgemäße Verfahren kann z. Bsp. bei der Untersuchung der Druckfestigkeit von Beton angewendet werden. Nach der oben beschriebenen Kalibrierung der Schall-Sonden 15.1 und 15.2 und dem Abgleich durch die Auswerteeinheit 17, wird Beton in das Aufnahmeelement 18 gefüllt. Danach wird 28 Tage abgewartet, um die 28-Tage-Druckfestigkeit des Betons durch Laufzeitmessung von Schall und durch Auswertung dieser Laufzeitmessung in der Auswerteeinheit 17 zu ermitteln. Nach einer vorgegebenen Zeitspanne, beispielsweise einem Jahr, kann nochmals eine Messung durchgeführt werden, um eine weitere Druckfestigkeit zu ermitteln. Das Schwinden des Betons wird durch Nachfließen von Gel und dem Nachdrücken der Membran 12. 1 bzw. 12.2 beim Schwinden des Betons ausgeglichen.

**Bezugszeichenliste**

| | | | | | |
|---|---|---|---|---|---|
| 1 | Wand | 34 | | 67 | |
| 2 | Wand | 35 | | 68 | |
| 3 | Distanzhülse | 36 | | 69 | |
| 4 | Schraubenbolzen | 37 | | 70 | |
| 5 | Rändelmutter | 38 | | 71 | |
| 6 | Stufenausnehmung | 39 | | 72 | |
| 7 | Führung | 40 | | 73 | |
| 8 | Schieber | 41 | | 74 | |
| 9 | Trichterschacht | 42 | | 75 | |
| 10 | Kammer | 43 | | 76 | |
| 11 | Platte | 44 | | 77 | |
| 12 | Membran | 45 | | 78 | |
| 13 | Rändelmutter | 46 | | 79 | |
| 14 | Schraubenbolzen | 47 | | | |
| 15 | Schall-Sonde | 48 | | | |
| 16 | Verbindung | 49 | | | |
| 17 | Auswerteeinheit | 50 | | a | Abstand |
| 18 | Aufnahmeelement | 51 | | P | Vorrichtung |
| 19 | | 52 | | | |
| 20 | | 53 | | | |
| 21 | | 54 | | | |
| 22 | | 55 | | | |
| 23 | | 56 | | | |
| 24 | | 57 | | | |
| 25 | | 58 | | | |
| 26 | | 59 | | | |
| 27 | | 60 | | | |
| 28 | | 61 | | | |
| 29 | | 62 | | | |
| 30 | | 63 | | | |
| 31 | | 64 | | | |
| 32 | | 65 | | | |
| 33 | | 66 | | | |

## Patentansprüche

1. Vorrichtung zur in situ Charakterisierung der Qualitätsparameter und/oder der Eigenschaften von anorganischen Bindemittelsystemen, insbesondere von aushärtbaren Gemischen, wobei sich das Bindemittelsystem in einem Aufnahmeelement (18) befindet, dem zumindest eine Wand (1, 2) mit einer Sonde (15.1, 15.2) zugeordnet ist, wobei zwischen dem Bindemittelsystem und der Sonde (15.1, 15.2) eine Kammer (10.1, 10.2) für ein Kontaktmaterial vorgesehen ist und eine Membran (12.1, 12.2) das Bindemittelsystem von dem Kontaktmaterial trennt, wobei die Membran bei einem Schwinden des Bindemittelsystems diesem folgt,
**dadurch gekennzeichnet,**
**dass** die Membran mit dem Bindemittelsystem durch Kleben verbunden ist.

2. Vorrichtung nach Anspruch 1, **dadurch gekennzeichnet, dass** die Membran zum Bindemittelsystem hin mit einem doppelseitigen Klebeband belegt ist.

## Claims

1. Device for the in situ characterisation of the quality parameters and/or the properties of inorganic binder systems, in particular of hardenable mixtures, wherein the binder system is located in a receiving element (18) to which at least one wall (1, 2) is connected by a probe (15.1, 15.2), wherein a chamber (10.1, 10.2) for a contact material is provided between the binder system and the probe (15.1, 15.2) and a membrane (12.1, 12.2) separates the binder system from the contact material, wherein the membrane follows the binder system as it shrinks,
**characterized in that**
the membrane is connected to the binder system by adhesive bonding.

2. The device according to claim 1, **characterised in that** the membrane is covered towards the binder system with a double-sided adhesive tape.

## Revendications

1. Dispositif destiné à la caractérisation in situ des paramètres de qualité et/ou des propriétés de systèmes de liant inorganiques, en particulier de mélanges durcissables, dans lequel le système de liant se trouve dans un élément de réception (18) auquel est associée au moins une paroi (1, 2) avec une sonde (15.1, 15.2), dans lequel est prévue, entre le système de liant et la sonde (15.1, 15.2), une chambre (10.1, 10.2) pour un matériau de contact, et une membrane (12.1, 12.2) sépare le système de liant du matériau de contact, dans lequel, lors du retrait du système de liant, la membrane suit ce dernier,
**caractérisé par le fait**
**que** la membrane est liée au système de liant par collage.

2. Dispositif selon la revendication 1, **caractérisé par le fait que** la membrane est recouverte du côté du système de liant d'un ruban adhésif double face.
